# EUROPÄISCHE PATENTSCHRIFT

(11) **EP 2 379 482 B1**
(45) Veröffentlichungstag und Bekanntmachung des Hinweises auf die Patenterteilung: **26.08.2015**
(21) Anmeldenummer: 09799604.5
(22) Anmeldetag: 15.12.2009
(51) Int. Cl.: C07C 67/08, C07C 69/00, C07C 69/80, B01D 3/36

(54) **HERSTELLUNG VON CARBONSÄUREESTERN UNTER STRIPPEN MIT ALKOHOL-DAMPF**
PRODUCTION OF CARBOXYLIC ACID ESTERS BY STRIPPING WITH ALCOHOL VAPOR
PRODUCTION D'ESTERS D'ACIDE CARBOXYLIQUE PAR STRIPPAGE AVEC DE LA VAPEUR D'ALCOOL

(30) Priorität: 16.12.2008 EP 08171795
(43) Veröffentlichungstag der Anmeldung: 26.10.2011
(73) Patentinhaber: BASF SE, 67056 Ludwigshafen (DE)
(72) Erfinder: PETERS, Jarren, 68163 Mannheim (DE); DISTELDORF, Walter, 67157 Wachenheim (DE); FRIESE, Katrin, 68199 Mannheim (DE); SCHÄFER, Thomas, 68165 Mannheim (DE); BEY, Oliver, 67150 Niederkirchen (DE)
(86) Internationale Anmeldenummer: PCT/EP2009/067179
(87) Internationale Veröffentlichungsnummer: WO 2010/076194

(56) Entgegenhaltungen:
- EP-A1- 0 434 390
- EP-A2- 0 334 154
- DE-A1- 2 503 195
- US-A1- 2004 106 813

## Beschreibung

Die vorliegende Erfindung betrifft ein Verfahren zur Herstellung von Carbonsäureestern durch Umsetzung eines Reaktionsgemisches, das eine Carbonsäure und/oder ein Carbonsäureanhydrid und einen Alkohol umfasst.

Ester der Phthalsäure, Adipinsäure, Sebacinsäure oder Maleinsäure finden weite Anwendung in Lackharzen, als Bestandteile von Anstrichmitteln und insbesondere als Weichmacher für Kunststoffe.

Es ist bekannt, Carbonsäureester durch Umsetzung von Carbonsäuren mit Alkoholen herzustellen. Diese Reaktion kann autokatalytisch oder katalytisch, beispielsweise durch Brönstedt- oder Lewissäuren, durchgeführt werden. Unabhängig von der Art der Katalyse entsteht immer ein temperaturabhängiges Gleichgewicht zwischen den Einsatzstoffen (Carbonsäure und Alkohol) und den Produkten (Ester und Wasser).

Die Umsetzung von inneren Carbonsäureanhydriden mit Alkoholen verläuft in zwei Schritten: Die Alkoholyse des Anhydrids zum Monoester verläuft in der Regel rasch und vollständig. Die weitere Umsetzung des Monoesters zum Diester unter Bildung von Reaktionswasser ist reversibel und verläuft langsam. Dieser zweite Schritt ist der geschwindigkeitsbestimmende Schritt der Reaktion.

Um das Gleichgewicht zu Gunsten des Esters (bzw. des Vollesters bei mehrbasigen Säuren) zu verschieben, wird in der Regel ein Schleppmittel eingesetzt, mit dessen Hilfe das Reaktionswasser aus dem Ansatz entfernt wird. Wenn einer der Einsatzstoffe (Alkohol oder Carbonsäure) niedriger siedet als der gebildete Ester und mit Wasser eine Mischungslücke bildet, kann ein Edukt als Schleppmittel verwendet und nach Abtrennung von Wasser wieder in den Ansatz zurückgeführt werden. Bei der Veresterung von höheren aliphatischen Carbonsäuren, aromatischen Carbonsäuren oder von zwei- oder mehrbasigen Carbonsäuren ist in der Regel der eingesetzte Alkohol das Schleppmittel. Dient der eingesetzte Alkohol als Schleppmittel, geht man üblicherweise so vor, dass man den Brüden aus dem Reaktor zumindest teilweise kondensiert, das Kondensat in eine wässrige Phase und eine im Wesentlichen aus dem zur Veresterung eingesetzten Alkohol bestehende organische Phase trennt und die organische Phase zumindest teilweise in den Reaktor zurückführt.

Die DE 2 503 195 A1 offenbart ein Verfahren zur kontinuierlichen Veresterung von Fettsäuren mit Alkoholen im Gegenstrom in Gegenwart eines Katalysators. Die Reaktion wird in zwei Stufen durchgeführt und in die zweite Stufe wird reiner Alkoholdampf eingeleitet. Bei dem Alkoholdampf, der in die zweite Stufe eingeleitet wird, handelt es sich um überhitzen Alkoholdampf.

Die EP 0 434 390 A1 offenbart ein Verfahren zur Veresterung einer Carbonsäure mit einem Alkohol bei erhöhter Temperatur und erhöhtem Druck. Ein gasförmiges und ein flüssiges Edukt werden kontinuierlich in einen Reaktor eingeführt. In einer Ausführungsform legt man eine Monocarbonsäure in einem Autoklaven als Flüssigkeit vor und führt Alkoholdampf durch oder über die Flüssigkeit. Als geeignete flüchtige Alkohole werden Methanol, Ethanol, Propanol, Isopropanol, ein Butanol, ein Pentanol oder 2-Ethylhexanol genannt. Bevorzugt liegt die Reaktionstemperatur im Bereich von 200 bis 260 °C und der Druck im Bereich von 0,5 bis 1,5 MPa.

Die EP 0 334 154 A2 offenbart ein Verfahren zur kontinuierlichen Veresterung von C₂-C₂₄-Fettsäuren mit C₁-C₅-Monoalkanolen oder C₂-C₃-Dialkanolen in flüssiger Phase bei erhöhten Temperaturen in Gegenwart von Katalysatoren, wobei man die Alkanole dampfförmig oberhalb des Kolonnensumpfes an den untersten Boden einer mit einer Mehrzahl von Böden ausgestatteten Reaktionskolonnne gibt. In Beispiel 1 und Beispiel 2 ist der Einsatz von Rein-Isopropanol bei 140°C und einem Kopfdruck der Reaktionskolonne von 800 hPa beschrieben.

Die US 2004/0106813 A1 offenbart ein Verfahren zur Veresterung einer Fettsäure. Das Verfahren umfasst einen Verfahrensschritt in dem man ein alkoholreiches Strippgas im Sumpf einer Kolonne durch Verdampfen erzeugt. In einem weiteren Verfahrensschritt führt man das alkoholreiche Strippgas in Gegenstrom durch eine katalytisch aktive Reaktionszone, um Wasser aus der Reaktionszone auszustrippen. Im Beispiel 2 wird Isopropanol als Alkohol bei einer Temperatur von 192 °C im Sumpf der Kolonne und einer Temperatur von 140°C in der Reaktionszone bei einem Druck von ca. 5100 mbar eingesetzt.

Die EP-A 1 186 593 beschreibt ein Verfahren zur Herstellung von Carbonsäureestern durch Reaktion von Di- oder Polycarbonsäuren oder deren Anhydride mit Alkoholen, wobei das Reaktionswasser durch azeotrope Destillation mit dem Alkohol entfernt wird. Die durch die azeotrope Destillation aus der Reaktion entfernte Flüssigkeitsmenge wird vollständig oder teilweise mit dem Alkohol wieder ergänzt.

H. Suter in Chemie-Ing.-Technik 41 (1969), Nr. 17 S. 971-974 beschreibt die kontinuierliche Herstellung von Phthalsäureestern in einer Rührkesselkaskade.

Der Stand der Technik enthält verschiedene Vorschläge, um die Entfernung des Reaktionswassers zu verbessern.

So beschreibt die EP 680 463 B1 ein Verfahren zur Veresterung von Säuren oder Säureanhydriden mit einem Monoalkohol oder einer Polyhydroxyverbindung, wobei man ein Reaktionsgemisch zum Sieden erhitzt und das Wasser als Dampf entfernt, wobei das Reaktionsgemisch kontinuierlich so gemischt wird, dass mindestens 2,5 Volumen Reaktionsgemisch intern pro Minute zirkuliert werden. Durch das Mischen unter den genannten Bedingungen soll die Umwandlungsrate erhöht werden.

Die EP-A 835 860 betrifft ein Verfahren zur Abtrennung von Wasser aus Reaktionsgemischen zur Veresterung von Säuren bzw. Säureanhydriden mit Alkoholen bei Siedetemperatur des Reaktionsgemisches, wobei das am niedrigsten siedende Edukt zunächst in unterstöchiometrischer Menge eingesetzt wird, das entstehende Dampfgemisch aus vorwiegend Wasser und der am niedrigsten siedenden Komponente an einer Membran entwässert wird, das entwässerte Dampfgemisch in das Reaktionsgemisch zurückgeführt und das am niedrigsten siedende Edukt im Laufe der Reaktion dem Reaktionsgemisch nachgesetzt wird.

Der Erfindung liegt die Aufgabe zugrunde, ein alternatives Verfahren zur Verbesserung der Abtrennung des Reaktionswassers bereitzustellen. Der Erfindung liegt insbesondere die Aufgabe zugrunde, ein Verfahren zur Herstellung von Estern mit niedriger Säurezahl bereitzustellen.

Die Aufgabe wird gelöst durch ein Verfahren zur Herstellung von Carbonsäureestern durch Umsetzung eines Reaktionsgemisches, das eine Carbonsäure und/oder ein Carbonsäureanhydrid und einen Alkohol umfasst, in Gegenwart eines Veresterungskatalysators, der unter Alkoholaten, Carboxylaten und Chelatverbindungen von Titan, Zirkonium, Zinn, Aluminium und Zink ausgewählt ist, in einem aus einem oder mehreren Rührkesselreaktoren bestehenden Reaktionssystem, wobei man Reaktionswasser als Alkohol-Wasser-Azeotrop mit dem Brüden abdestilliert, den Brüden aus wenigstens einem Reaktor zumindest teilweise kondensiert, das Kondensat in eine wässrige Phase und eine Alkoholphase trennt und die Alkoholphase zumindest teilweise in das Reaktionssystem zurückführt, dadurch gekennzeichnet, dass man das Reaktionsgemisch mit überhitztem Alkohol-Dampf behandelt.

Überhitzter Alkohol-Dampf zeichnet sich dadurch aus, dass seine Temperatur oberhalb des thermodynamisch definierten Taupunkts bei Betriebsdruck liegt. Als Alkohol-Dampf verwendet man den Alkohol in gasförmigem Zustand, der die Alkoholkomponente des Reaktionsgemisches ist. Die Temperatur des Alkohol-Dampfs ist vorzugsweise wenigstens 20 °C höher als der Taupunkt.

Das Reaktionsgemisch wird zweckmäßigerweise so mit dem Alkohol-Dampf behandelt, dass eine große Austauschfläche zwischen dem flüssigen Reaktionsgemisch und dem Alkohol-Dampf, vorzugsweise unter turbulenten Bedingungen, geschaffen wird. Die Behandlung mit Alkohol-Dampf während der Umsetzung hat einen Stripp-Effekt und vervollständigt die Entfernung des Reaktionswassers. Über den Alkohol-Dampf wird außerdem Energie in das Reaktionssystem eingetragen; der Energieeintrag über den Reaktormantel kann gedrosselt werden. Dadurch kann eine Überhitzung des Reaktionsgemisches in der Nähe des Reaktormantels und die Bildung von Nebenprodukten verringert werden.

Zur Behandlung eignen sich beispielsweise alle gängigen Apparaturen zum Strippen von Flüssigkeiten mit Gasen.

In bevorzugten Ausführungsformen wird der Alkohol-Dampf unter der Flüssigkeitsoberfläche in das siedende Reaktionsgemisch eingeführt, so dass er das Reaktionsgemisch durchperlt. Der Druck des Alkohol-Dampfs muss ausreichend hoch sein, um den hydrostatischen Druck des Reaktionsgemisches oberhalb der Alkohol-Dampf-Einspeisung zu überwinden. Z. B. kann man den Alkohol-Dampf 20 bis 50 cm unterhalb der Flüssigkeitsoberfläche des Reaktionsgemisches einführen.

Der Alkohol-Dampf kann über beliebige geeignete Vorrichtungen eingespeist werden. Es eignen sich z. B. Begasungslanzen, die fest installiert sein können, oder vorzugsweise Düsen. Die Düsen können am oder in der Nähe des Reaktorbodens vorgesehen sein. Die Düsen können hierzu als Öffnungen einer den Reaktor umgebenden Hohlkammer ausgebildet sein. Bevorzugt werden jedoch Tauchdüsen mit geeigneten Zuleitungen eingesetzt. Mehrere Düsen können z. B. in Form eines Kranzes angeordnet sein. Die Düsen können nach oben oder nach unten weisen. Die Düsen weisen vorzugsweise schräg nach unten.

Vorzugsweise wird das Reaktionsgemisch durchmischt, um einen Austausch von Reaktionsgemisch im Reaktorbereich unterhalb der Alkohol-Dampf-Einspeisung mit Reaktionsgemisch im Reaktorbereich oberhalb der Alkohol-Dampf-Einspeisung zu bewirken. Zur Vermischung eignen sich beispielsweise Rührer oder eine Umlaufpumpe.

Der Alkohol-Dampf wird vorzugsweise durch Verdampfen von flüssigem, wasserfreien Alkohol erzeugt. Die Erzeugung des Alkohol-Dampfs kann mit einem beliebigen Dampferzeuger erfolgen, z. B. einem Dampfkessel, Plattenverdampfer, Rohrverdampfer oder Rohrbündelverdampfer. Platten- und Rohrbündelverdampfer und Kombinationen davon sind im Allgemeinen bevorzugt.

Die eingeführte Alkohol-Dampf-Menge unterliegt keinen besonderen Beschränkungen und beträgt bei kontinuierlicher Verfahrensführung z. B. 0,01 bis 0,5 kg/h, insbesondere 0,05 bis 0,2 kg/h pro kg/h Reaktionsgemisch.

Unter "Reaktionssystem" wird ein Reaktor oder eine Anordnung von mehreren Reaktoren verstanden. Mehrere Reaktoren sind vorzugsweise hintereinander geschaltet. Das erfindungsgemäße Verfahren kann diskontinuierlich oder kontinuierlich durchgeführt werden, wird aber vorzugsweise kontinuierlich durchgeführt.

Erfindungsgemäß werden Rührkesselreaktoren verwendet. Die Rührkesselreaktoren bestehen meist aus metallischen Werkstoffen, wobei Edelstahl bevorzugt ist. Der Reaktionsansatz wird vorzugsweise mit Hilfe eines Rührers oder einer Umlaufpumpe intensiv vermischt.

Wenngleich das erfindungsgemäße Verfahren auch mit nur einem Rührkessel betrieben werden kann, ist es bei kontinuierlicher Verfahrensführung für eine möglichst vollständige Umsetzung zweckmäßig, mehrere Reaktoren miteinander in Form einer Kaskade zu verbinden. Die einzelnen Reaktoren werden vom Reaktionsgemisch nacheinander durchlaufen, wobei der Ablauf des ersten Reaktors dem zweiten Reaktor, der Ablauf des zweiten Reaktors dem dritten Reaktor usw. zugeführt wird. Die Kaskade kann z. B. 2 bis 10 Reaktoren umfassen, wobei 3 bis 6 Reaktoren bevorzugt sind. Carbonsäure und/oder Carbonsäureanhydrid und Alkohol werden kontinuierlich in den ersten Reaktor zugegeben.

Während der Reaktion wird ein Alkohol-Wasser-Gemisch als Azeotrop aus der Reaktionsmischung abdestilliert. Während der Reaktion wird außerdem Alkohol in den Reaktor bzw. die einzelnen Reaktoren des Reaktionssystems nachgespeist. In die Reaktoren, in die Alkohol-Dampf eingeführt wird, kann die Zugabe zusätzlichen Alkohols unterbleiben; gegebenenfalls kann zusätzlich flüssiger Alkohol zugegeben werden. In die Reaktoren, in die kein Alkohol-Dampf eingeführt wird, wird vorzugsweise flüssiger Alkohol nachgespeist.

Wenn das Reaktionssystem eine Kaskade von mehreren Reaktoren umfasst, führt man in das Reaktionsgemisch in wenigstens einem Reaktor, vorzugsweise zumindest in das Reaktionsgemisch im letzten Reaktor, Alkohol-Dampf ein. In den Reaktoren einer Kaskade nimmt der Umsatzgrad vom ersten bis zum letzten Reaktor stetig zu. Das erfindungsgemäße Strippen mit Alkohol-Dampf unterstützt vor allem in den späteren Reaktoren einer Kaskade die Entfernung der noch verbliebenen geringen Mengen Reaktionswasser.

Wenn mehr als ein Reaktor mit Alkohol-Dampf behandelt wird, kann der Alkohol-Dampf parallel zu den einzelnen Reaktoren geführt werden oder der Alkohol-Dampf passiert mehrere Reaktoren nacheinander. Es sind auch Kombinationen denkbar, in denen zwei oder mehrere Reaktoren mit frischem Alkohol-Dampf durchperlt werden und der Dampf aus wenigstens einem der Reaktoren durch wenigstens einen weiteren Reaktor geleitet wird. Bei der parallelen Versorgung mit Alkohol-Dampf ist jeder zu durchperlende Reaktor über eine Alkohol-Dampf-Leitung mit dem Alkohol-Verdampfer verbunden.

Wenn der Alkohol-Dampf mehrere Reaktoren hintereinander passiert, geht man so vor, dass man den Brüden aus einem Reaktor, in den Alkohol-Dampf eingeführt wird, sammelt und den Brüden dampfförmig in das Reaktionsgemisch in wenigstens einem der vorausgehenden Reaktoren einführt. Der frische Alkohol-Dampf muss mit ausreichendem Druck eingeführt werden, um den kumulierten hydrostatischen Druck des Reaktionsgemisches in den nacheinander zu durchlaufenden Reaktoren zu überwinden. In diesem Fall ist das Druckgefälle zwischen den Reaktoren ausreichend, damit der gesammelte Brüden das Reaktionsgemisch im vorausgehenden Reaktor durchperlen kann. Andernfalls kann man den gesammelten Brüden verdichten, bevor dieser in den vorausgehenden Reaktor eingeführt wird. Beispielsweise kann man in einer Kaskade von sechs Reaktoren frischen Alkohol-Dampf in das Reaktionsgemisch im letzten Reaktor einführen, den Brüden aus dem letzten Reaktor sammeln und dampfförmig in das Reaktionsgemisch im fünften Reaktor einführen, den Brüden aus dem fünften Reaktor sammeln und dampfförmig in das Reaktionsgemisch im vierten Reaktor einführen.

Im Allgemeinen kondensiert man den Brüden aus wenigstens einem Reaktor zumindest teilweise, trennt das Kondensat in eine wässrige Phase und eine Alkoholphase und führt die Alkoholphase zumindest teilweise in das Reaktionssystem zurück. "Rückführung in das Reaktionssystem" bedeutet, dass die Alkoholphase in wenigstens einen beliebigen Reaktor des Reaktionssystems geleitet wird.

Zur Kondensation bzw. partiellen Kondensation des Brüden können alle geeigneten Kondensatoren verwendet werden. Diese können mit beliebigen Kühlmedien gekühlt werden. Kondensatoren mit Luftkühlung und/oder Wasserkühlung sind bevorzugt, wobei die Luftkühlung besonders bevorzugt ist.

Das erhaltene Kondensat wird einer Phasentrennung in eine wässrige Phase und eine organische Phase unterzogen. Üblicherweise wird das Kondensat hierzu in einen Phasenscheider (Dekanter) geleitet, wo es durch mechanisches Absetzen in zwei Phasen zerfällt, die getrennt abgezogen werden können. Die wässrige Phase wird abgetrennt und kann, gegebenenfalls nach Aufarbeitung, verworfen oder als Strippwasser bei der Nachbehandlung des Esters verwendet werden.

Der Brüden aus den einzelnen Reaktoren einer Kaskade kann vereinigt werden und gemeinsam kondensiert werden. Gegebenenfalls kann man jeweils mehrere Reaktoren der Kaskade zu einer Untereinheit zusammenfassen, wobei dann jeweils die Untereinheiten mit einem Kondensator gekoppelt sind. Es besteht daneben weiterhin die Möglichkeit, jeden Reaktor der Kaskade mit einem Kondensator zu koppeln.

Die zurückzuführende Alkoholphase kann in einen beliebigen Reaktor einer Kaskade geleitet oder auf mehrere Reaktoren der Kaskade aufgeteilt werden. Es ist jedoch bevorzugt, die zurückzuführende Alkoholphase nicht in den letzten Reaktor der Kaskade zu leiten. Vorzugsweise leitet man die zurückzuführende Alkoholphase ausschließlich oder überwiegend in den ersten Reaktor der Kaskade.

Für die Rückführung der Alkoholphase in das Reaktionssystem gibt es verschiedene Möglichkeiten. Eine Möglichkeit ist, die organische Phase, gegebenenfalls nach Erwärmen, in das flüssige Reaktionsgemisch zu pumpen.

Zur thermischen Optimierung des Verfahrens führt man die Alkoholphase aber vorzugsweise über eine Kolonne (so genannte Rückalkohol-Kolonne) in das Reaktionssystem zurück, in der man der rückgeführten Alkoholphase zumindest einen Teil des Brüden entgegenführt. Zweckmäßigerweise führt man die Alkoholphase am Kopf oder im oberen Bereich in die Rückalkohol-Kolonne ein. Das ablaufende Kondensat der Rückalkohol-Kolonne gelangt in das Reaktionssystem zurück, bei Verwendung einer Reaktorkaskade vorzugsweise in den ersten Reaktor. Die Rückführung der Alkoholphase über die Rückalkohol-Kolonne weist den Vorteil auf, dass die rückgeführte Alkoholphase vorerwärmt und von Wasserspuren befreit wird, die nach der Phasentrennung in der organischen Phase verblieben sind bzw. gemäß ihrer thermodynamischen Löslichkeit in der organischen Phase gelöst sind. Bei der Rückalkohol-Kolonne kann es sich beispielsweise um eine Bodenkolonne, Packungskolonne oder Füllkörperkolonne handeln. Eine geringe Trennstufenzahl ist im Allgemeinen ausreichend. Geeignet ist z. B. eine Kolonne mit 2 bis 10 theoretischen Trennstufen.

Bei Verwendung einer Reaktorkaskade verlässt der Brüden vorzugsweise zumindest den ersten Reaktor über die Rückalkohol-Kolonne. Ein oder mehrere oder alle weiteren Reaktoren können ebenfalls einen Brüdenabzug zur Rückalkohol-Kolonne aufweisen.

Das erfindungsgemäße Verfahren ist prinzipiell auf alle Veresterungen anwendbar, bei denen das Reaktionswasser als Azeotrop mit einem Alkohol destillativ abgetrennt wird.

Im erfindungsgemäßen Verfahren werden als Säurekomponente Carbonsäuren oder Carbonsäureanhydride eingesetzt. Bei mehrbasigen Carbonsäuren können auch teilweise anhydridisierte Verbindungen eingesetzt werden. Ebenso ist es möglich, Gemische aus Carbonsäuren und Anhydriden zu verwenden.

Die Säuren können aliphatisch, einschließlich carbocyclisch, heterocyclisch, gesättigt oder ungesättigt, sowie aromatisch, einschließlich heteroaromatisch, sein.

Zu den geeigneten Carbonsäuren zählen aliphatische Monocarbonsäuren mit wenigsten 5 Kohlenstoffatomen, insbesondere 5 bis 20 Kohlenstoffatomen, wie n-Pentansäure, 2-Methylbuttersäure, 3-Methylbuttersäure, 2-Methylpentansäure, 2-Ethylbuttersäure, n-Heptansäure, 2-Methylhexansäure, Isoheptansäuren, Cyclohexancarbonsäure, n-Octansäure, 2-Ethylhexansäure, Isooctansäuren, n-Nonansäure, 2-Methyloctansäure, Isononansäuren, n-Decansäure, Isodecansäuren, 2-Methylundecansäure, Isoundecansäure, Tricyclodecancarbonsäure und Isotridecancarbonsäure.

Weiter eignen sich aliphatische C₄-C₁₀-Dicarbonsäuren bzw. deren Anhydride, wie z. B. Maleinsäure, Fumarsäure, Maleinsäureanhydrid, Bernsteinsäure, Bernsteinsäureanhydrid, Adipinsäure, Korksäure, Trimethyladipinsäure, Azelainsäure, Decandisäure, Dodecandisäure, Brassylsäure. Beispiele für carbocyclische Verbindungen sind: 1,2-Cyclohexandicarbonsäure (Hexahydrophthalsäure), 1,2-Cyclohexandicarbonsäureanhydrid (Hexahydrophthalsäureanhydrid), Cyclohexan-1,4-dicarbonsäure, Cyclohex-4-en-1,2-dicarbonsäure, Cyclohexen-1,2-dicarbonsäureanhydrid, 4-Methylcyclohexan-1,2-dicarbonsäure, 4-Methylcyclohexan-1,2-dicarbonsäureanhydrid, 4-Methylcyclohex-4-en-1,2-dicarbonsäure, 4-Methylcyclohex-4-en-1,2-dicarbonsäureanhydrid.

Beispiele geeigneter aromatischer Dicarbonsäuren bzw. deren Anhydride sind: Phthalsäure, Phthalsäureanhydrid, Isophthalsäure, Therephthalsäure, oder Naphthalindicarbonsäuren und deren Anhydride.

Beispiele geeigneter aromatischer Tricarbonsäuren (bzw. Anhydride) sind Trimellitsäure, Trimellitsäureanhydrid oder Trimesinsäure; Ein Beispiel einer geeigneten aromatischen Tetracarbonsäure bzw. ihres Anhydrids sind Pyromellitsäure und Pyromellitsäureanhydrid.

Besonders bevorzugt wird im erfindungsgemäßen Verfahren Phthalsäureanhydrid als Carbonsäurekomponente eingesetzt.

Im erfindungsgemäßen Verfahren werden bevorzugt verzweigte oder lineare aliphatische Alkohole mit 4 bis 13 C-Atomen eingesetzt. Die Alkohole sind einwertig und können sekundär oder primär sein.

Die eingesetzten Alkohole können aus verschiedenen Quellen stammen. Geeignete Einsatzstoffe sind beispielsweise Fettalkohole, Alkohole aus dem Alfolprozess oder Alkohole oder Alkoholgemische, die durch Hydrierung von gesättigten oder ungesättigten Aldehyden gewonnen wurden, insbesondere solchen, deren Synthese einen Hydroformylierungsschritt einschließt.

Alkohole, die im erfindungsgemäßen Verfahren eingesetzt werden, sind beispielsweise n-Butanol, Isobutanol, n-Octanol(1), n-Octanol(2), 2-Ethylhexanol, Nonanole, Decylalkohole oder Tridecanole hergestellt durch Hydroformylierung oder Aldolkondensation und anschließende Hydrierung. Die Alkohole können als reine Verbindung, als Gemisch isomerer Verbindungen oder als Gemisch von Verbindungen mit unterschiedlicher C-Zahl eingesetzt werden. Ein bevorzugtes Beispiel eines derartigen Alkoholgemisches ist ein C₉/C₁₁-Alkoholgemisch.

Bevorzugte Einsatzalkohole sind Gemische isomerer Octanole, Nonanole oder Tridecanole, wobei die letzteren aus den entsprechenden Butenoligomeren, insbesondere Oligomeren von linearen Butenen, durch Hydroformylierung und anschließender Hydrierung gewonnen werden können. Die Herstellung der Butenoligomeren kann im Prinzip nach drei Verfahren durchgeführt werden. Die sauer katalysierte Oligomerisierung, bei der technisch z. B. Zeolithe oder Phosphorsäure auf Trägem eingesetzt werden, liefert die verzweigtesten Oligomere. Bei Einsatz von linearen Butenen entsteht beispielsweise eine C₈-Fraktion, die im Wesentlichen aus Dimethylhexenen besteht (WO 92/13818). Ein ebenfalls weltweit ausgeübtes Verfahren ist die Oligomerisierung mit löslichen Ni-Komplexen, bekannt als DIMERSOL-Verfahren (B. Cornils, W. A. Herrmann, Applied Homogenous Catalysis with Organometallic Compounds, Seite 261-263, Verlag Chemie 1996). Weiterhin wird die Oligomerisierung an Nickel-FestbettKatalysatoren ausgeübt, wie beispielsweise der OCTOL-Process (Hydrocarbon Process., Int. Ed. (1986) 65 (2. Sect. 1), Seite 31-33) oder das Verfahren gemäß der WO 95/14647 oder WO 01/36356.

Ganz besonders bevorzugte Einsatzstoffe für die erfindungsgemäße Veresterung sind Gemische isomerer Nonanole oder Gemische isomerer Tridecanole, die durch Oligomerisierung linearer Butene zu C₈-Olefinen und C₁₂-Olefinen nach dem Octol-Prozess oder gemäß der WO 95/14647, mit anschließender Hydroformylierung und Hydrierung hergestellt werden.

Weiterhin eignen sich Alkylenglykolmonoether, insbesondere Ethylenglykolmonoether, wie Ethylenglykolmonomethylether, Ethylenglykolmonoethylether und Ethylenglykolmonobuthylether; und Polyalkylenglykolmonoether insbesondere Polyethylenglykolmonoether, wie Polyethylenglykolmonomethylether.

Besonders bevorzugte Alkohohole sind 2-Ethylhexanol, 2-Propylheptanol, Isononanol-Isomerengemische, Decanol-Isomerengemische und C₉/C₁₁-Alkoholgemische.

Die erfindungsgemäße Veresterung wird in Gegenwart eines Veresterungskatalysators durchgeführt.

Geeigneterweise ist der Veresterungskatalysator unter Alkoholaten, Carboxylaten und Chelatverbindungen von Titan, Zirkonium, Zinn, Aluminium und Zink ausgewählt. Es eignen sich Tetraalkyltitanate, wie Tetramethyltitanat, Tetraethyltitanat, Tetra-n-propyltitanat, Tetra-isopropyltitanat, Tetra-n-butyltitanat, Tetra-isobutyltitanat, Tetra-sec-butyltitanat, Tetraoctyltitanat, Tetra-(2-ethylhexyl)-titanat; Dialkyltitanate ((RO)₂TiO₂, worin R z.B. für iso-Propyl, n-Butyl, iso-Butyl steht), wie Isopropyl-n-butyltitanat; Titan-Acetylacetonat-Chelate, wie Di-isopropoxy-bis(acetylacetonat)titanat, Di-isopropoxy-bis(ethylacetylacetonat)titanat, Di-n-butyl-bis(acetylacetonat)titanat, Di-n-butyl-bis(ethylacetoacetat)titanat, Tri-isopropoxid-bis(acetylacetonat)titanat; Zirkontetraalkylate, wie Zirkontetraethylat, Zirkontetrabutylat, Zirkontetrabutyrat, Zirkontetrapropylat, Zirkoncarboxylate, wie Zirkondiacetat; Zirkon--Acetylacetonat-Chelate, wie Zirkontetra(acetylacetonat), Tributoxyzirkonacetylacetonat, Dibutoxyzirkon(bis-acetylacetonat); Aluminiumtrisalkylate, wie Aluminiumtriisopropylat, Aluminiumtrisbutylat; Aluminium-Acetylacetonat-Chelate, wie Aluminiumtris(acetylacetonat) und Aluminiumtris(ethylacetylacetonat). Insbesondere werden Isopropyl-n-butyltitanat, Tetra(isopropyl)orthotitanat oder Tetra(butyl)orthotitanat eingesetzt.

Die Katalysatorkonzentration hängt von der Art des Katalysators ab. Bei den bevorzugt eingesetzten Titanverbindungen beträgt diese 0,005 bis 1,0 Gew.-% bezogen auf das Reaktionsgemisch, insbesondere 0,01 bis 0,3 Gew.-%.

Bei diskontinuierlicher Verfahrensdurchführung können die Edukte und der Katalysator gleichzeitig oder nacheinander in den Reaktor eingefüllt werden. Der Katalysator kann in reiner Form oder als Lösung, bevorzugt gelöst in einem der Einsatzstoffe, zu Beginn oder erst nach Erreichen der Reaktionstemperatur eingebracht werden. Carbonsäureanhydride reagieren häufig mit Alkoholen bereits autokatalytisch, d. h. unkatalysiert zu den entsprechenden Estercarbonsäuren (Halbestern), beispielsweise Phthalsäureanhydrid zum Phthalsäuremonoester. Daher ist ein Katalysator häufig erst nach dem ersten Reaktionsschritt erforderlich.

Bei kontinuierlicher Verfahrensdurchführung führt man Ströme der Edukte und des Katalysators in den Reaktor bzw. bei Verwendung einer Reaktorkaskade in den ersten Reaktor der Kaskade ein. Die Verweilzeit im Reaktor bzw. den einzelnen Reaktoren wird dabei durch das Volumen der Reaktoren und den Mengenstrom der Edukte bestimmt.

Der umzusetzende Alkohol, der als Schleppmittel dient, kann im stöchiometrischen Überschuss, bevorzugt 30 bis 200 %, besonders bevorzugt 50 bis 100 % der stöchiometrisch notwendigen Menge eingesetzt werden.

Die Reaktionstemperaturen liegen zwischen 160 °C und 270 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen unter Bildung von Olefinen oder Bildung farbiger Nebenprodukte. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktor eingestellt werden. Bei niedrig siedenden Alkoholen kann daher die Umsetzung bei Überdruck und bei höher siedenden Alkoholen bei vermindertem Druck durchgeführt werden. Beispielsweise wird bei der Umsetzung von Phthalsäureanhydrid mit einem Gemisch isomerer Nonanole in einem Temperaturbereich von 170 °C bis 250 °C im Druckbereich von 200 mbar bis 3 bar gearbeitet.

Alle Reaktoren einer Kaskade können bei gleicher Temperatur betrieben werden. Im Allgemeinen ist es aber bevorzugt, die Temperatur vom ersten zum letzten Reaktor einer Kaskade stetig zu erhöhen, wobei ein Reaktor bei gleicher oder höherer Temperatur betrieben wird, als der in Fließrichtung des Reaktionsgemisches stromaufwärts gelegene Reaktor. Zweckmäßigerweise können alle Reaktoren bei im Wesentlichen gleichem Druck betrieben werden.

Nach Beendigung der Reaktion enthält das Reaktionsgemisch, das im Wesentlichen aus dem gewünschten Ester und überschüssigem Alkohol besteht, neben dem Katalysator und/oder dessen Folgeprodukte geringe Mengen an Estercarbonsäure(n) und/oder nicht umgesetzter Carbonsäure.

Zur Aufarbeitung dieser Esterrohgemische wird der überschüssige Alkohol entfernt, die saueren Verbindungen neutralisiert, der Katalysator zerstört und die dabei entstandenen festen Nebenprodukte abgetrennt. Dabei wird der größte Teil des nicht umgesetzten Alkohols bei Normaldruck oder im Vakuum abdestilliert. Die letzten Spuren des Alkohols können z. B. durch Wasserdampfdestillation, insbesondere im Temperaturbereich von 120 bis 225 °C unter Vakuum, entfernt werden. Die Abtrennung des Alkohols kann als erster oder als letzter Aufarbeitungsschritt erfolgen.

Die Neutralisation der saueren Stoffe, wie Carbonsäuren, Estercarbonsäuren oder gegebenenfalls der saueren Katalysatoren, erfolgt durch Zugabe von Basen, z. B. von Alkali- und/oder Erdalkalimetallcarbonaten, -hydrogencarbonaten oder -hydroxiden. Das Neutralisationsmittel kann in fester Form oder bevorzugt als Lösung, insbesondere als wässrige Lösung eingesetzt werden. Hier wird häufig Natronlauge einer Konzentration von 1 bis 30 Gew.-%, bevorzugt von 20 bis 30 Gew.-% verwendet. Das Neutralisationsmittel wird in einer Menge zugesetzt, die dem Einfachen bis dem Vierfachen, insbesondere dem Einfachen bis Zweifachen der stöchiometrisch notwendigen Menge, die durch Titration bestimmt wird, entspricht.

Die so hergestellten Ester aus mehrbasischen Carbonsäuren, wie beispielsweise Phthalsäure, Adipinsäure, Sebacinsäure, Maleinsäure, und aus Alkoholen, finden weite Anwendung in Lackharzen, als Bestandteile von Anstrichmitteln und insbesondere als Weichmacher für Kunststoffe. Geeignete Weichmacher für PVC sind Dioctylphthalate, Diisononylphthalate, Diisodecylphthalate und Dipropylheptylphthalate.

Die Erfindung wird durch die beigefügte Zeichnung und die folgenden Beispiele näher erläutert.

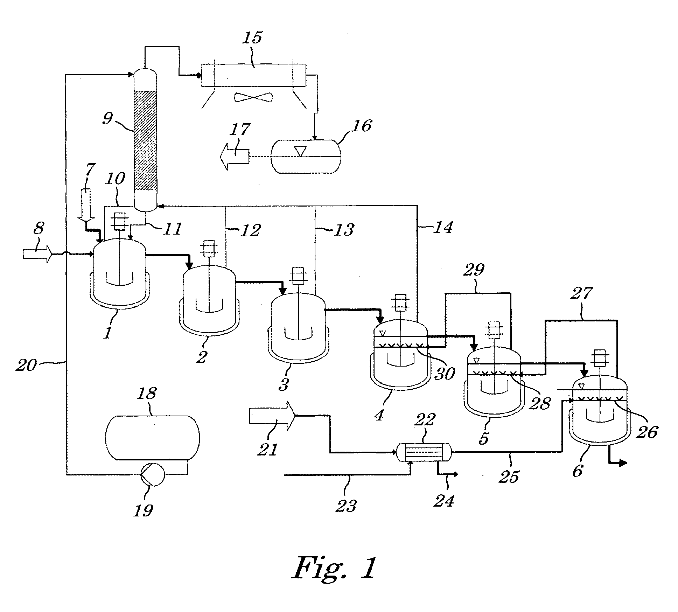

Fig. 1 zeigt eine zur Durchführung des erfindungsgemäßen Verfahrens geeignete Anlage. Die Anlage umfasst eine Kaskade aus sechs Rührkesseln 1, 2, 3, 4, 5 und 6, wobei der Ablauf des ersten Kessels dem zweiten Kessel, der Ablauf des zweiten Kessels dem dritten Kessel usw. zugeführt wird. Über eine Alkohol-Sammelleitung (nicht dargestellt) wird über Zuleitungen Alkohol in die Rührkessel 1, 2, 3, 4 und 5 dosiert. Über die Leitung 7 wird eine Säurekomponente, beispielsweise Phthalsäureanhydrd (PSA), in den ersten Kessel 1 gespeist. In den ersten Kessel 1 wird über die Leitung 8 Veresterungskatalysator zugesetzt.

Die aus dem ersten Kessel 1 aufsteigenden Brüden werden über die Leitung 10 abgezogen und gelangen in die Rückalkoholkolonne 9; der Rücklauf aus der Rückalkohol-Kolonne 9 gelangt über die Leitung 11 in den ersten Kessel 1. Die Brüdenabzüge 12, 13, 14 aus dem zweiten, dritten und vierten Kessel 2, 3, 4 führen ebenfalls zur Rückalkohol-Kolonne 9.

Die vereinten Brüden werden einem Kondensator 15, z. B. einem luftgekühlten Kondensator, zugeführt. Der aus dem Kondensator 15 austretende gemischtphasige Strom wird im Phasenscheider 16 getrennt. Die untere, wässrige Phase wird über eine Leitung (nicht dargestellt) abgezogen und verworfen. Die obere, organische Phase wird über die Leitung 17 dem Rückalkohol-Sammelgefäß 18 zugeleitet. Ein Teil der organischen Phase kann zur Vermeidung der Aufpegelung von Nebenprodukten ausgeschleust werden oder behandelt, z. B. aufgereinigt, und dem Rückalkohol-Sammelgefäß 18 zugeleitet werden.

Über die Pumpe 19 und die Leitung 20 wird Alkohol aus dem Rückalkohol-Sammelgefäß 18 am Kopf oder in den oberen Bereich der Rückalkohol-Kolonne 9 eingespeist, wo er den aufsteigenden Brüden entgegengeführt wird, und gelangt über die Leitung 11 in den ersten Kessel 1.

Über die Leitung 21 wird Alkohol dem Verdampfer 22, z. B einem Rohrbündelverdampfer, zugeführt und verdampft. Der Verdampfer 22 wird mit Heißdampf beheizt, der über die Leitung 23 herangeführt wird. Das Heißdampf-Kondensat wird über die Leitung 24 abgeführt. Der erzeugte Alkohol-Dampf wird über die Leitung 25 und den Düsenkranz 26 unterhalb der Flüssigkeitsoberfläche in das Reaktionsgemisch im Kessel 6 eingeführt. Der Alkohol-Dampf durchperlt das Reaktionsgemisch; der Stripp-Effekt unterstützt die Abtrennung des Reaktionswassers als Alkohol-Wasser-Azeotrop. Die Brüden im Gasraum des Kessels 6 werden über die Leitung 27 gesammelt und über den Düsenkranz 28 unterhalb der Flüssigkeitsoberfläche in das Reaktionsgemisch im Kessel 5 eingeführt. Die Druckdifferenz zwischen dem Kessel 6 und dem Kessel 5 ist ausreichend, dass die Brüden aus dem Kessel 6 ohne zusätzliche Verdichtung den hydrostatischen Druck des Reaktionsgemisches oberhalb des Düsenkranzes 28 im Kessel 5 überwinden. Die Brüden im Gasraum des Kessels 5 werden über die Leitung 29 gesammelt und über den Düsenkranz 30 unterhalb der Flüssigkeitsoberfläche in das Reaktionsgemisch im Kessel 4 eingeführt.

### BEISPIELE

### Vergleichsbeispiel 1: Herstellung von Diisononylphthalat

Zur kontinuierlichen Herstellung von Diisononylphthalat (DINP) verwendete man eine Kaskade von sechs Rührkesseln. In jeden Reaktionskessel wurde Isononanol zudosiert, insgesamt 731 g/h Isononanol. In den ersten Reaktionskessel wurden 0,3 g/h Propyltitanat zudosiert. Außerdem wurden 358 g/h Phthalsäureanhydrid (PSA) in den ersten Reaktionskessel dosiert. Über eine Rückalkohol-Kolonne am ersten Rührkessel wurden außerdem etwa 665 g/h Isononanol-Kreislaufrückstrom als Rücklauf auf die Rückalkohol-Kolonne gegeben.

Die Brüden aus dem ersten Rührkessel wurden über die Rückalkohol-Kolonne abgezogen, deren Rücklauf in den ersten Rührkessel zurückgeleitet wurde. Der Brüdenabzug aus dem zweiten bis dritten Rührkessel erfolgte ebenfalls über die Rückalkohol-Kolonne; die Brüden aus dem vierten bis sechsten Rührkessel wurden direkt abgezogen.

Die Brüden aus der Veresterung wurden in einem Luftkühler kondensiert und das Kondensat auf eine Temperatur von 70 °C gekühlt. In einem Phasenscheider wurden die organische und wässrige Phase bei Normaldruck getrennt. Das Wasser wurde ausgeschleust; ein Teil der organischen Phase einem Alkoholsammelbehälter zugeführt.

Das aus dem letzten Rührkessel ablaufende Esterrohgemisch wurde aufgearbeitet, indem der überschüssige Alkohol entfernt, die saueren Verbindungen neutralisiert, der Katalysator zerstört und die dabei entstandenen festen Nebenprodukte abgetrennt wurden. Man erhielt 1000 g/h DINP mit einer Säurezahl von 0,5 mg KOH/g.

### Beispiel 1

Die kontinuierliche Herstellung von DINP erfolgte analog zum Vergleichsbeispiel 1, wobei jedoch in den sechsten Rührkessel 30 cm unterhalb der Flüssigkeitsoberfläche des Reaktionsgemisches Isononanol-Dampf eingeführt wurde, der durch Verdampfen von 105 g/h Isononanol in einem Verdampfer erzeugt wurde und die Zugabe von flüssigem Isononanol in diesen Kessel ersetzte. Die Brüden aus dem sechsten Kessel wurden unterhalb der Flüssigkeitsoberfläche des Reaktionsgemisches in den fünften Kessel, die Brüden aus dem fünften Kessel unterhalb der Flüssigkeitsoberfläche des Reaktionsgemisches in den vierten Kessel geleitet.

Die Säurezahl des erhaltenen DINP war mehr als 80% niedriger als im Vergleichsbeispiel 1; die Raumzeitausbeute stieg um mehr als 30 %.

## Patentansprüche

1. Verfahren zur Herstellung von Carbonsäureestern durch Umsetzung eines Reaktionsgemisches, das eine Carbonsäure und/oder ein Carbonsäureanhydrid und einen Alkohol umfasst, in Gegenwart eines Veresterungskatalysators, der unter Alkoholaten, Carboxylaten und Chelatverbindungen von Titan, Zirkonium, Zinn, Aluminium und Zink ausgewählt ist, in einem aus einem oder mehreren Rührkesselreaktoren bestehenden Reaktionssystem, wobei man Reaktionswasser als Alkohol-Wasser-Azeotrop mit dem Brüden abdestilliert, den Brüden aus wenigstens einem Reaktor zumindest teilweise kondensiert, das Kondensat in eine wässrige Phase und eine Alkoholphase trennt und die Alkoholphase zumindest teilweise in das Reaktionssystem zurückführt, **dadurch gekennzeichnet, dass** man das Reaktionsgemisch mit überhitztem Alkohol-Dampf behandelt.

2. Verfahren nach Anspruch 1, wobei man den Alkohol-Dampf unterhalb der Flüssigkeitsoberfläche des Reaktionsgemisches einführt und das Reaktionsgemisch mit dem Alkohol-Dampf durchperlt.

3. Verfahren nach Anspruch 2, wobei man das Reaktionsgemisch durchmischt, um einen Austausch von Reaktionsgemisch im Reaktorbereich unterhalb der Alkohol-Dampf-Einspeisung mit Reaktionsgemisch im Reaktorbereich oberhalb der Alkohol-Dampf-Einspeisung zu bewirken.

4. Verfahren nach Anspruch 2 oder 3, wobei das Reaktionssystem eine Kaskade von mehreren Reaktoren umfasst und man in das Reaktionsgemisch in mehr als einem Reaktor Alkohol-Dampf einführt.

5. Verfahren nach einem der vorhergehenden Ansprüche, wobei das Reaktionssystem eine Kaskade von mehreren Reaktoren umfasst und man zumindest in das Reaktionsgemisch im letzten Reaktor Alkohol-Dampf einführt.

6. Verfahren nach Anspruch 4, wobei man zumindest den Brüden aus dem letzten Reaktor sammelt und dampfförmig in das Reaktionsgemisch in wenigstens einem der vorausgehenden Reaktor einführt.

7. Verfahren nach einem der vorhergehenden Ansprüche, wobei man die Alkoholphase über eine Kolonne in das Reaktionssystem zurückführt, in der man der rückgeführten Alkoholphase zumindest einen Teil des Brüden entgegenführt.

8. Verfahren nach Anspruch 7, wobei das Reaktionssystem eine Kaskade von mehreren Reaktoren umfasst und man die Alkoholphase ausschließlich oder überwiegend in den ersten Reaktor der Kaskade zurückführt.

9. Verfahren nach einem der vorhergehenden Ansprüche, wobei die Carbonsäure unter aliphatischen Monocarbonsäuren mit wenigsten 5 Kohlenstoffatomen, aliphatischen C₄-C₁₀-Dicarbonsäuren, aromatischen Monocarbonsäuren, aromatischen Dicarbonsäuren, aromatischen Tricarbonsäuren, aromatischen Tetracarbonsäuren und Anhydriden davon ausgewählt ist.

10. Verfahren nach einem der vorhergehenden Ansprüche, wobei der Alkohol unter C₄-C₁₃-Alkoholen, Alkylenglykolmonoethern und Polyalkylenglykolmonoethern und Gemischen davon ausgewählt ist.

## Claims

1. A process for preparing carboxylic esters by reaction of a reaction mixture comprising a carboxylic acid and/or a carboxylic anhydride and an alcohol in the presence of an esterification catalyst selected from among alkoxides, carboxylates and chelate compounds of titanium, zirconium, tin, aluminum and zinc, in a reaction system comprising one or more stirred tank reactors, with water of reaction being distilled off as alcohol-water azeotrope with the vapor, where the vapor from at least one reactor is at least partly condensed, the condensate is separated into an aqueous phase and an alcohol phase and the alcohol phase is at least partly recirculated to the reaction system, wherein the reaction mixture is treated with superheated alcohol vapor.

2. The process according to claim 1, wherein the alcohol vapor is introduced below the surface of the liquid reaction mixture and the alcohol vapor bubbles through the reaction mixture.

3. The process according to claim 2, wherein the reaction mixture is mixed in order to effect exchange of reaction mixture in the reactor region below the introduction of alcohol vapor with reaction mixture in the reactor region above the introduction of alcohol vapor.

4. The process according to claim 2 or 3, wherein the reaction system comprises a cascade of a plurality of reactors and alcohol vapor is introduced into the reaction mixture in more than one reactor.

5. The process according to any of the preceding claims, wherein the reaction system comprises a cascade of a plurality of reactors and alcohol vapor is introduced at least into the reaction mixture in the last reactor.

6. The process according to claim 4, wherein at least the vapor from the last reactor is collected and introduced in vapor form into the reaction mixture in at least one of the preceding reactors.

7. The process according to any of the preceding claims, wherein the alcohol phase is recirculated to the reaction system via a column in which the recirculated alcohol phase is conveyed in countercurrent to at least part of the vapor.

8. The process according to claim 7, wherein the reaction system comprises a cascade of a plurality of reactors and the alcohol phase is recirculated exclusively or predominantly into the first reactor of the cascade.

9. The process according to any of the preceding claims, wherein the carboxylic acid is selected from among aliphatic monocarboxylic acids having at least 5 carbon atoms, aliphatic C₄-C₁₀-dicarboxylic acids, aromatic monocarboxylic acids, aromatic dicarboxylic acids, aromatic tricarboxylic acids, aromatic tetracarboxylic acids and anhydrides thereof.

10. The process according to any of the preceding claims, wherein the alcohol is selected from among C₄-C₁₃-alcohols, alkylene glycol monoethers and polyalkylene glycol monoethers and mixtures thereof.

## Revendications

1. Procédé pour la production d'esters d'acides carboxyliques par mise en réaction d'un mélange réactionnel qui comprend un acide carboxylique et/ou un anhydride d'acide carboxylique et un alcool, en présence d'un catalyseur d'estérification qui est choisi parmi des alcoolates, carboxylate et composés chélate de titane, zirconium, étain, aluminium et zinc, dans un système réactionnel consistant en un ou plusieurs réacteurs de type cuve à agitation, dans lequel on élimine par distillation avec la vapeur l'eau de réaction sous forme d'azéotrope alcool-eau, la vapeur provenant d'au moins un réacteur est au moins partiellement condensée, on fractionne le condensat en une phase aqueuse et une phase alcoolique et on recycle au moins en partie la phase alcoolique dans le système réactionnel, **caractérisé en ce qu'**on traite le mélange réactionnel par de la vapeur d'alcool surchauffée.

2. Procédé selon la revendication 1, dans lequel on introduit la vapeur d'alcool au-dessous de la surface du liquide du mélange réactionnel et le mélange réactionnel est traversé par la vapeur d'alcool en fines bulles.

3. Procédé selon la revendication 2, dans lequel on homogénéise le mélange réactionnel afin de provoquer un échange du mélange réactionnel dans la zone du réacteur au-dessous de l'alimentation en vapeur d'alcool avec le mélange réactionnel dans la zone du réacteur au-dessus de l'alimentation en vapeur d'alcool.

4. Procédé selon la revendication 2 ou 3, dans lequel le système réactionnel comprend une cascade de plusieurs réacteurs et dans plus d'un réacteur on introduit la vapeur d'alcool dans le mélange réactionnel.

5. Procédé selon l'une quelconque des revendications précédentes, dans lequel le système réactionnel comprend une cascade de plusieurs réacteurs et au moins dans le dernier réacteur on introduit de la vapeur d'alcool dans le mélange réactionnel.

6. Procédé selon la revendication 4, dans lequel on recueille au moins la vapeur du dernier réacteur et on l'introduit sous forme de vapeur dans le mélange réactionnel dans au moins un des réacteurs précédents.

7. Procédé selon l'une quelconque des revendications précédentes, dans lequel on renvoie la phase alcoolique dans le système réactionnel via une colonne dans laquelle on fait passer la phase alcoolique recyclée à contre-courant d'au moins une partie de la vapeur.

8. Procédé selon la revendication 7, dans lequel le système réactionnel comprend une cascade de plusieurs réacteurs et on recycle la phase alcoolique exclusivement ou principalement dans le premier réacteur de la cascade.

9. Procédé selon l'une quelconque des revendications précédentes, dans lequel l'acide carboxylique est choisi parmi des acides monocarboxyliques aliphatiques ayant au moins 5 atomes de carbone, des acides dicarboxyliques aliphatiques en C₄-C₁₀, des acides monocarboxyliques aromatiques, des acides dicarboxyliques aromatiques, des acides tricarboxyliques aromatiques, des acides tétracarboxyliques aromatiques et des anhydrides de ceux-ci.

10. Procédé selon l'une quelconque des revendications précédentes, dans lequel l'alcool est choisi parmi des alcools en C₄-C₁₃, des monoéthers d'alkylèneglycols et des monoéthers de polyalkylène-glycols et des mélanges de ceux-ci.
